# EUROPEAN PATENT SPECIFICATION

(11) **EP 3 030 245 B1**
(45) Date of publication and mention of the grant of the patent: **04.10.2023**
(21) Application number: 14833801.5
(22) Date of filing: 07.08.2014
(51) Int. Cl.: A61K 31/718, A23L 33/00, A61P 3/06, A61P 3/10, A61P 5/50, A23L 29/212, A61K 36/81, A23L 33/21

(54) **IMPROVEMENT OF BLOOD LIPIDS, GLUCOSE TOLERANCE AND INSULIN SENSITIVITY**
VERBESSERUNG DES BLUTFETTWERTES, DER GLUCOSETOLERANZ UND DER INSULINSENSITIVITÄT
AMÉLIORATION DE LIPIDES SANGUINS, DE TOLÉRANCE AU GLUCOSE ET DE SENSIBILITÉ À L'INSULINE

(30) Priority: 07.08.2013 US 201361863004 P
(43) Date of publication of application: 15.06.2016
(73) Proprietor: Mcpharma Biotech Inc., Carberry, Manitoba R0K 0H0 (CA)
(72) Inventor: MCLAREN, Derek, Carberry, Manitoba R0K 0H0 (CA); MCLAREN, Earl, Carberry, Manitoba R0K 0H0 (CA)
(74) Representative: FARAGO Patentanwälte GmbH
(86) International application number: PCT/CA2014/050740
(87) International publication number: WO 2015/017934

(56) References cited:
- CHEN YA-YEN ET AL: "Sweet Potato [Ipomoea batatas (L.) Lam. "Tainong 57"] Starch Improves Insulin Sensitivity in High-Fructose Diet-Fed Rats by Ameliorating Adipocytokine Levels, Pro-Inflammatory Status, and Insulin Signaling", JOURNAL OF NUTRITIONAL SCIENCE AND VITAMINOLOGY, UNIVERSITY OF TOKYO PRESS, TOKYO, JP, vol. 59, no. 4, 1 August 2013 (2013-08-01) , pages 272-280, XP009193120, ISSN: 0301-4800
- DING Y-Q ET AL: "Correlation of resistant starch with blood glucose and lipid of type 2 diabetic rats", CHINESE JOURNAL OF CLINICAL REHABILITATION, ZHONGGUO KANGFU YIXUEHUI, CN, vol. 9, no. 15, 21 April 2005 (2005-04-21) , pages 92-93, XP009193150, ISSN: 1671-5926
- KENDALL CYRIL W C ET AL: "Resistant starches and health", JOURNAL OF AOAC INTERNATIONAL, AOAC INTERNATIONAL, ARLINGTON, VA, US, vol. 87, no. 3, 1 May 2004 (2004-05-01), pages 769-774, XP009193119, ISSN: 1060-3271
- Hubert W Lopez ET AL: "Nutrient Interactions and Toxicity Class 2 Resistant Starches Lower Plasma and Liver Lipids and Improve Mineral Retention in Rats", , 1 January 2000 (2000-01-01), XP55336515, Retrieved from the Internet: URL:http://jn.nutrition.org/content/131/4/ 1283.full.pdf#page=1&view=FitH [retrieved on 2017-01-18]
- TRINIDAD, T.P. ET AL.: 'Sweet potato and cassava can modify cholesterol profile in humans with moderately raised serum cholesterol levels' FOOD AND NUTRITION SCIENCES vol. 4, May 2013, pages 491 - 495, XP055314742
- BRONKOWSKA, M. ET AL.: 'Effect of resistant starch RS4 added to the high-fat diets on selected biochemical parameters in wistar rats' ROCZNIKI PANSTWOWEGO ZAKLADU HIGIENY vol. 64, no. 1, March 2013, pages 19 - 24, XP055314744 Retrieved from the Internet: <URL:wydawnictwa.pzh.gov.pl/roczniki_pzh/po bierz-artykul?id=960>
- RABEN, A. ET AL.: 'Resistant starch: the effect on postprandial glycemia, hormonal response, and satiety' AMERICAN JOURNAL OF CLINICAL NUTRITION vol. 60, 1994, pages 544 - 551, XP055299892
- ROBERTSON, M.D. ET AL.: 'Insulin-sensitizing effects of dietary resistant starch and effects on skeletal muscle and adipose tissue metabolism' AMERICAN JOURNAL OF CLINICAL NUTRITION vol. 82, 2005, pages 559 - 567, XP055314746
- JOHNSTON, K.L. ET AL.: 'Resistant starch improves insulin sensitivity in metabolic syndrome' DIABETIC MEDICINE vol. 27, 2010, pages 3 91 - 397, XP055314747
- ROBERTSON, M.D. ET AL.: 'Insulin-sensitizing effects on muscle and adipose tissue after dietary fiber intake in men and women with metabolic syndrome' THE JOURNAL OF CLINICAL ENDOCRINOLOGY AND METABOLISM vol. 97, no. 9, 2012, pages 3326 - 3332, XP055314748
- NUGENT, A.P.: 'Health properties of resistant starch' NUTRITION BULLETIN vol. 30, 2005, pages 27 - 54, XP055251394

## Description

### PRIOR APPLICATION INFORMATION

The instant application claims the benefit of United States Provisional Patent Application Serial Number 61/863,004, filed August 7, 2013.

### BACKGROUND OF THE INVENTION

Resistant starch (RS) is defined as the sum of starch and starch digestion products that are not digested in the small intestine but instead reach the large intestine as a fermentable fiber substrate. Previous research has established RS as an effective dietary prebiotic supplement to modulate intestinal function and improve systemic health in both animals and humans. In human health and disease prevention, RS has potential application in weight management, the treatment of gastrointestinal disorders, and the improvement of blood lipids, glucose tolerance and insulin sensitivity [1, 2].

It is important to note however that all resistant starch is not equal. Specifically, there is exceptional diversity encountered among RS varieties. Specifically, RS varieties originating from different plant sources and manufactured with alternative processing technologies will possess unique physiochemical properties.

Although dietary guidelines recommend a daily dietary fiber intake of 25-35 g, it is clear that fiber consumption amongst Canadians is low. Canadians consume between 3-8 g of RS per day, partly due to the variability of RS content in common foods and a general lack of commercially available RS-enriched foods and nutraceutical products.

Chen, Ya-Yen et al. (Journal of Nutritional Science and Vitaminology, 2013, 59 (4), pages 272-280) relates to the effects of starch in high-fructose diet fed rats. Chen et al. uses sweet potato as the source of high GI starch which is a different plant not belonging to the Solanum family of potatoes.

Ding, Y.-Q. et al. (Chinese Journal of Clinical Rehabilitation, 2005, 9 (15), pages 92-93) describes the results on diabetes in rats fed resistant starch and digestible starch by gastric perfusion, however, the type of resistant potato starch is not disclosed.

Kendall, Cyril W. et al. (Journal of AOAC International, 2004, 87 (3), pages 769-774) discusses the role of resistant starch for health and tested highly digestible corn starch and two levels of high amylose resistant corn starch. Kendall et al. teaches that truly resistant starches do not improve carbohydrate tolerance of the next meal. However, Kendall et al. is silent on potato starch.

Lopez, H.W. et al. (Journal of Nutrition, 2001, 131(4) pages 1283 to 1289) describes that rats were fed a raw potato containing diet (RPS) and a high amylose corn starch-containing diet (HAS). However, Lopez et al. does not teach or suggest reduction of blood glucose or insulin resistance. Furthermore, Lopez et al. teaches that triglycerides are reduced compared to control, in contrast to the increase in HDL plasma levels as taught in the present invention.

Trinidad, T.P. et al. (Food and Nutrition Sciences, 2013, 4, pages 491-495) investigates sweet potato and cassava for modification on human cholesterol levels but is silent on potatoes.

Bronkowska, M. et al. (Roczniki Panstwowego Zakladu Higieny, 64 (1), pages 19 to 24) studied the effect of RS4 resistant starch added to the high-fat diets on selected biochemical parameters in Wistar rats. Bronkowska uses RS4 resistant starch that has been modified chemically through esterification with phosphoric salts. However, D7 is silent on a raw, unmodified RS2 potato starch.

Raben, A. et al. (American Journal of Clinical Nutrition 1994, 60, pages 544-551) studied the effect of restitant starch on postprandial glycemia, hormonal response, and satiety. Raben et al. discloses a study, whereby subjects were fed with a carbohydrate-rich diet for three days prior to being fed two test-meals - one comprising 50 g raw potato starch (R) and one containing 50 g pregelatinized starch (S). However, D7 is silent on a regimen using unmodified resistant potato starch.

In order for dietary fibers such as RS to have a significant and sustained public health impact, there is a need to develop novel strategies to increase dietary fiber intake. Development of a RS capsule may be a convenient and effective approach to increase RS consumption and improve human health. Surprisingly, there are currently no such commercially available RS-based fiber capsules available within Canada.

### SUMMARY OF THE INVENTION

According to the invention, there is provided a native, unmodified RS type 2 resistant potato starch having a resistant starch content of for at least 60% for use according to claim 1. Preferably, the resistant potato starch is MSPrebiotic^{®} Resistant Potato Starch.

In one aspect of the invention, there is provided the resistant potato starch of the invention for use in the treatment of a human for increasing High Density Lipoprotein plasma levels in an individual in need of such treatment. Preferably, the resistant potato starch is MSPrebiotic^{®} Resistant Potato Starch.

In yet another embodiment of the invention, there is provided the resistant potato starch of the invention for use in the preparation of a medicament for increasing High Density Lipoprotein plasma levels in a human in need of such treatment. Preferably, the resistant potato starch is MSPrebiotic^{®} Resistant Potato Starch.

For example, in certain embodiments, a human in need of such treatment may be a human with a fasting HDL plasma level below 40 mg/dL or below 1 mmol/L or a human with a fasting LDL to HDL ratio of greater than 5 or a human with high fasting LDL levels combined with low fasting HDL levels or a human with a familial history of cardiovascular disease or who is otherwise considered to be at risk of developing cardiovascular disease.

According to one aspect of the invention, the resistant potato starch of the invention is used for decreasing blood glucose levels in a human in need of such treatment comprising administering to said human an effective amount of resistant potato starch. Preferably, the resistant potato starch is MSPrebiotic^{®} Resistant Potato Starch.

In yet another embodiment of the invention, the resistant potato starch of the invention is used for the preparation of a medicament for decreasing blood glucose levels in an individual in need of such treatment. Preferably, the resistant potato starch is MSPrebiotic^{®} Resistant Potato Starch.

As will be appreciated by one of skill in the art, a human in need of such treatment in these embodiments may be for example a human who has or is at risk of having a blood glucose level outside the normal range, that is, greater than between 70 and 100 mg/dL, or greater than 100 mg/dL. Alternatively, the human may be a human who has diabetes and who has or is suspected of having or is at risk of having a blood glucose range for outside of 70-130 mg/dL or greater than 130 mg/dL before meals and less than 180 mg/dL after meals. Alternatively, the human in need of such treatment may be hyperglycemic or chronically hyperglycemic or may be diabetic.

According to one aspect of the invention, the resistant potato starch of the invention is used for decreasing insulin resistance in a human in need of such treatment comprising administering to said human an effective amount of resistant potato starch. Preferably, the resistant potato starch is MSPrebiotic^{®} Resistant Potato Starch.

In another embodiment of the invention, there is provided use of resistant potato starch to decrease insulin resistance in an individual in need of such treatment. Preferably, the resistant potato starch is MSPrebiotic^{®} Resistant Potato Starch.

In yet another embodiment of the invention, there resistant potato starch of the invention is used for the preparation of a medicament for decreasing insulin resistance in an individual in need of such treatment. Preferably, the resistant potato starch is MSPrebiotic^{®} Resistant Starch.

For example, in these embodiments, a human in need of such treatment may be a human with increased insulin resistance, for example a human with Type 2 diabetes or a person with a familial history of Type 2 diabetes or a person at risk of developing Type 2 diabetes.

### BRIEF DESCRIPTION OF THE DRAWINGS

Figure 1: Time course of body weight gain (lbs).
Figure 2: Blood Lipid Response (mmol/L).
Figure 3: HDL Particle Number (µmol/L).
Figure 4: Blood glucose (% change)
Figure 5: Blood insulin (µIU/mL)
Figure 6: HOMA-IR
Figure 7: VLDL Particle Number (nmol/L)
Figure 8: Total LDL Particle Number (nmol/L)
Figure 9: Lipoprotein Particle Size (nm)

### DESCRIPTION OF THE PREFERRED EMBODIMENTS

Unless defined otherwise, all technical and scientific terms used herein have the same meaning as commonly understood by one of ordinary skill in the art to which the invention belongs. Although any methods and materials similar or equivalent to those described herein can be used in the practice or testing of the present invention, the preferred methods and materials are now described.

MSP Starch Products Inc., manufactures MSPrebiotic^{®} Resistant Potato Starch, a native, unmodified RS type 2 preparation of food grade quality for animal and human food application.

In order to evaluate the potential application of MSPrebiotic^{®} Resistant Potato Starch as a novel health promoting fiber vehicle in the human nutraceutical industry, a pre-clinical examination of the metabolic health benefits of a novel MSPrebiotic^{®} Resistant Potato Starch capsule in a domestic pig model fed a typical Western diet was undertaken.

Previous research in both humans and biomedical animal models suggests that resistant starch consumption may have potential application in weight management, the improvement of blood lipids, glucose tolerance and insulin sensitivity. However, clinical research also suggests that there is significant variability in the health promoting responses to RS consumption, partly due to the exceptional diversity that is encountered among RS varieties, as discussed herein.

The objective of this pre-clinical study was to access the metabolic responses to a novel MSPrebiotic^{®} Resistant Potato Starch capsule in a pig model. Twelve 8-week old male Yorkshire pigs were randomly assigned to two groups: 1) a placebo group supplemented with capsules containing normal gelatinized starch; or 2) a RS group supplemented with RS capsules containing MSPrebiotic^{®} Resistant Potato Starch at a dose of 10 g/d.

As discussed below, compared with the placebo group, MSPrebiotic^{®} Resistant Potato Starch supplementation increased (p<0.05) plasma total HDL-particles (28%) and reduced blood glucose (-11%) and insulin resistance (- 54%) as estimated by HOMA-IR.

High-Density Lipoprotein (HDL) is one of the five major groups of lipoproteins. Specifically, HDL is the smallest of the lipoprotein particles and transports cholesterol primarily to the liver. Furthermore, increasing one's HDL levels has been found to improve cardiovascular health. Specifically, individuals with low HDL-C levels, for example, lower than 40 mg/dL or about 1 mmol/L or individuals who have a ratio of LDL-C (Low Density Lipoprotein) to HDL-C of 5 or greater are considered to be at greater risk of developing cardiovascular diseases.

As discussed below, MSPrebiotic^{®} Resistant Potato Starch has been shown to increase the total number of HDL-particles in plasma compared to a control of similar age and fed an otherwise substantially similar diet by 28%.

Consequently, an effective amount of resistant potato starch, for example, MSPrebiotic^{®} Resistant Potato Starch, can be administered to a human in need of such treatment to increase HDL particle number.

Accordingto one aspect of the invention, the resistant potato starch of the invention is used for increasing High Density Lipoprotein plasma levels in a human in need of such treatment comprising administering to said individual an effective amount of resistant potato starch. Preferably, the resistant potato starch is MSPrebiotic^{®} Resistant Potato Starch.

As will be appreciated by one of skill in the art, the increase in HDL particle number or HDL plasma level in the human may be in comparison to the HDL particle number or HDL plasma level in said human prior to beginning administration or treatment. Alternatively, the increase may be in comparison to an untreated control of similar age and condition. As will be appreciated by one of skill in the art, the control does not necessarily need to be repeated every time.

Furthermore, the effective amount may vary according to many different factors, for example, the age, weight, and/or condition of the human. It is of note that the appropriate effective amount for a given human can be easily determined through routine experimentation.

For illustrative purposes, an "effective amount" for humans, may be 0.25 grams to 40 grams of MSPrebiotic^{®} Resistant Potato Starch. Alternatively, an effective amount may be 0.5 grams to 40 grams or 0.25 grams to 30 grams or 0.5 grams to 30 grams.

As will be appreciated by one of skill in the art, the "effective amount" may be taken on a regular schedule or regimen, for example, once per day or every other day.

It is important to note that the "effective amount" does not need to be taken in a single dose and may be taken in multiple or partial doses throughout the day, as discussed herein.

For example, convenient dosages of resistant starch include but are by no means limited to for example 250 mg capsules or tablets, 500 mg capsules or tablets, a teaspoon of resistant starch, a tablespoon of resistant starch and the like. As will be known by those of skill in the art, a "teaspoon" is typically considered to correspond to approximately 5 grams while a tablespoon is considered to correspond to approximately 10 grams. The resistant starch may be in the form of a powder. Other suitable dosages will be readily apparent to one of skill in the art.

it is noted that in some embodiments, the "effective amount" may be for example one or more teaspoon(s) of MSPrebiotic^{®} Resistant Potato Starch, for example, one, two, three or four teaspoon(s) MSPrebiotic^{®} Resistant Potato Starch. In some embodiments, this dosage may be taken on a regular schedule or regime, for example, once per day, twice per day, three times per day, four times per day, every other day or as needed or desired.

In yet other embodiments, the "effective amount" may be for example one or more tablespoon(s) of MSPrebiotic^{®} Resistant Potato Starch, for example, one, two or three tablespoon(s) MSPrebiotic^{®} Resistant Potato Starch. In some embodiments, this dosage may be taken on a regular schedule or regime, for example, once per day, twice per day, three times per day, four times per day, every other day or as needed or desired.

As discussed herein, other forms of resistant starch may be used within the invention, provided the product or medicament comprising the resistant starch, for example resistant potato starch, for example MSPrebiotic^{®} Resistant Potato Starch, is high in resistant starch. As used herein, a starch that has "high" resistant starch content is a starch that is at least 60% resistant starch.

According to the invention, the resistant potato starch is at least 60% resistant potato starch.

Yet further, the inventors have discovered that a key aspect in maintaining the integrity of the resistant starch, that is maintain the starch as a high resistant starch is maintaining the starch at a temperature below 60°C. As will be apparent to one of skill in the art, this includes production of the resistant starch itself and also preparation of medicaments such as tablets and capsules and functional foods and/or beverages to which the resistant starch is added.

For example, a human in need of such treatment may be a human with a fasting HDL plasma level below 40 mg/dL or below 1 mmol/L or a human with a fasting LDL to HDL ratio of greater than 5 or a human with high fasting LDL levels combined with low fasting HDL levels or a human with a familial history of cardiovascular disease or who is otherwise considered to be at risk of developing cardiovascular disease. The blood sugar concentration or blood glucose level is the amount of glucose present in blood. The mean normal level in humans is about 5.5 mM (5.5 mmol/L or 100 mg/dL). The normal blood glucose level for non-diabetics should be between 70 and 100 mg/dL. The blood glucose target range for diabetics should be 70-130 mg/dL before meals and less than 180 mg/dL after eating. Blood sugar levels that are persistently high are referred to as hyperglycemic and diabetes is characterized by persistent hyperglycemia.

As discussed herein, the treatment group fed MSPrebiotic^{®} Resistant Potato Starch demonstrated reduced blood glucose (-11%) compared to a control of similar age and fed an otherwise substantially similar diet.

Consequently, an effective amount of resistant potato starch, for example, MSPrebiotic^{®} Resistant Potato Starch is administered to a human in need of such treatment to decrease blood glucose.

As will be appreciated by one of skill in the art, the decrease in blood glucose levels in the human may be in comparison to the glucose levels in said human prior to beginning administration or treatment. Alternatively, the decrease may be in comparison to an untreated control of similar age and condition. As will be appreciated by one of skill in the art, the control does not necessarily need to be repeated every time.

Furthermore, the effective amount may vary according to many different factors, for example, the age, weight, and/or condition of the human. It is of note that the appropriate effective amount for a given human can be easily determined through routine experimentation.

In yet another aspect of the invention, there is provided of the MSPrebiotic^{®} Resistant Potato Starch for use in the treatment of a human for decreasing blood glucose levels in a human in need of such treatment.

In yet another aspect of the invention, there is provided the MSPrebiotic^{®} Resistant Potato Starch for use in the treatment of diabetes in a human in need of such treatment.

As will be appreciated by one of skill in the art, a human in need of such treatment may be for example a human who has or is at risk of having a blood glucose level outside the normal range, that is, greater than between 70 and 100 mg/dL, or greater than 100 mg/dL. Alternatively, the human may be a human who has diabetes and who has or is suspected of having or is at risk of having a blood glucose range for outside of 70-130 mg/dL or greater than 130 mg/dL before meals and less than 180 mg/dL after meals. Alternatively, the human in need of such treatment may be hyperglycemic or chronically hyperglycemic or may be diabetic.

Insulin resistance is a physiological condition in which cells fail to respond to the normal actions of insulin. Specifically, as a result of changes in their surface receptors, cells are unable to use insulin as effectively and beta cells in the pancreas increase their production of insulin which in turn leads to hyperglycemia. Insulin resistance is generally associated with Type 2 diabetes.

As discussed herein, the treatment group fed MSPrebiotic^{®} Resistant Potato Starch demonstrated a reduction in insulin resistance of 54% as estimated by HOMA-IR compared to the control group.

Consequently, an effective amount of resistant potato starch, for example, MSPrebiotic^{®} Resistant Potato Starch can be administered to a human in need of such treatment to decrease insulin resistance.

Accordingly, in one aspect of the invention, there is provided the resistant potato starch of the invention for decreasing insulin resistance in a human in need of such treatment comprising administering to said human an effective amount of resistant potato starch. Preferably, the resistant potato starch is MSPrebiotic^{®} Resistant Potato Starch.

As will be appreciated by one of skill in the art, the decrease in insulin resistance in the human may be in comparison to insulin resistance in said human prior to beginning administration or treatment. Alternatively, the decrease may be in comparison to an untreated control of similar age and condition. As will be appreciated by one of skill in the art, the control does not necessarily need to be repeated every time.

Furthermore, the effective amount may vary according to many different factors, for example, the age, weight, and/or condition of the human. It is of note that the appropriate effective amount for a given human can be easily determined through routine experimentation.

In another embodiment of the invention, there is provided the resistant potato starch of the invention for decreasing insulin resistance in a human in need of such treatment. Preferably, the resistant potato starch is MSPrebiotic^{®} Resistant Potato Starch.

For example, in these embodiments, a human in need of such treatment may be a human with increased insulin resistance, for example a human with Type 2 diabetes or a person with a familial history of Type 2 diabetes or a person at risk of developing Type 2 diabetes.

As discussed herein, these results indicate that resistant potato starch, for example, MSPrebiotic^{®} Resistant Potato Starch is an effective dietary supplement offering protection against multiple metabolic risk factors that are associated with cardiovascular disease and diabetes.

According to one embodiment of the invention, there is provided the resistant potato starch of the invention for use in the treatment of a human for increasing High Density Lipoprotein plasma levels in a human in need of such treatment comprising administering to said human 0.25 grams to 40 grams of MSPrebiotic^{®} Resistant Potato Starch daily.

For example, in these embodiments, a human in need of such treatment may be a human with a fasting HDL plasma level below 40 mg/dL or below 1 mmol/L or a human with a fasting LDL to HDL ratio of greater than 5 or a human with high fasting LDL levels combined with low fasting HDL levels or a human with a familial history of cardiovascular disease or who is otherwise considered to be at risk of developing cardiovascular disease.

According to one embodiment of the invention, the resistant potato starch is provided for decreasing blood glucose levels in a human in need of such treatment comprising administering to said human 0.25 grams to 40 grams of MSPrebiotic^{®} Resistant Potato Starch daily.

As will be appreciated by one of skill in the art, a human in need of such treatment in these embodiments may be for example a human who has or is at risk of having a blood glucose level outside the normal range, that is, greater than between 70 and 100 mg/dL, or greater than 100 mg/dL. Alternatively, the human may be a human who has diabetes and who has or is suspected of having or is at risk of having a blood glucose range for outside of 70-130 mg/dL or greater than 130 mg/dL before meals and less than 180 mg/dL after meals. Alternatively, the human in need of such treatment may be hyperglycemic or chronically hyperglycemic or may be diabetic.

According to one embodiment of the invention, there is provided a method of decreasing insulin resistance in a human in need of such treatment comprising administering to said human 0.25 grams to 40 grams of MSPrebiotic^{®} Resistant Potato Starch daily.

For example, in these embodiments, a human in need of such treatment may be a human with increased insulin resistance, for example a human with Type 2 diabetes or a person with a familial history of Type 2 diabetes or a person at risk of developing Type 2 diabetes.

Yet further, in the above embodiments, "daily" does not necessarily mean "every day" but may mean 70%, 80%, 90% or more of days within a given month or other suitable time period. As will be appreciated by one of skill in the art, many suitable products for administering resistant potato starch such as MSPrebiotic^{®} Resistant Potato Starch can be developed for example, a capsule in the nutraceutical industry as well as alternative food and/or beverage options that would more readily allow daily RS intakes in an effective amount, for example, as discussed herein.

It is of note that as discussed herein, although no statistically significant difference was observed between HDL-C concentrations, RS consumption increased HDL lipoprotein levels. For illustrative purposes, the lipoprotein can be referred to as the 'boat' while the HDL-C itself is the 'cargo'.

The invention will now be explained and illustrated by way of examples. However, the invention is not necessarily limited to the examples.

The pre-clinical assessment included the following metabolic parameters in response to dietary supplementation of MSPrebiotic^{®} Resistant Starch capsules for 30 days:
1. Standard fasting assessment of lipid biomarkers of cardiovascular disease risk including blood total cholesterol, low-density lipoprotein (LDL) cholesterol, high-density lipoprotein (HDL) cholesterol, and triglycerides (TAG).
2. Detailed fasting analysis of lipoprotein distribution patterns including very low-density lipoprotein (VLDL), LDL, and HDL particle number and size.
3. Blood glucose, insulin, and estimation of insulin resistance using homeostatic model assessment (HOMA).

### Animals

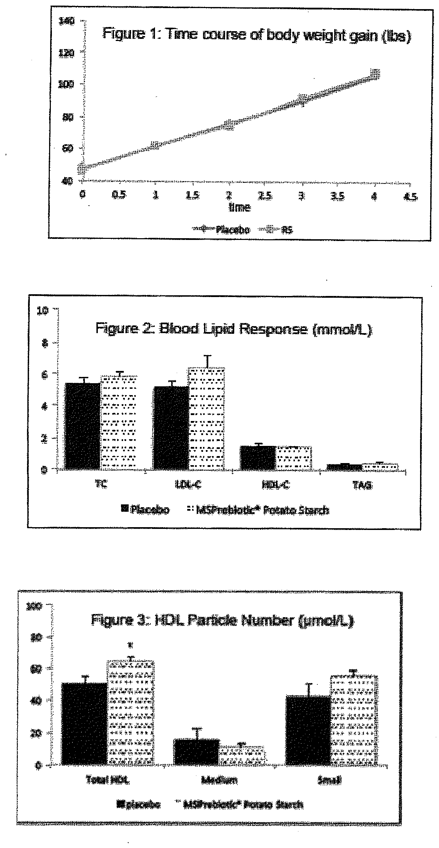

All animals remained healthy throughout the course of the experiment. No difference was observed in food intake or body weight gain (Figure 1) between the placebo and RS animals. The pigs readily consumed the capsules when mixed into the morning and evening powdered ration. For two pigs (one placebo, one RS) it was necessary to break open the capsules and feed the resistant potato starch as a free powder mixed in with the meal.

### Blood Lipid and Lipoprotein Response

We observed no difference (p<0.05) in standard fasting lipid biomarkers of cardiovascular disease risk between the placebo and RS-fed pigs (Figure 2). Although some studies have observed reductions in blood lipids in response to RS consumption, the majority of previous studies suggest that blood lipid responses to RS consumption are highly variable and dependent on the type and amount of RS and subject specific factors including gender and baseline lipid levels [4, 5].

As we are not aware of any previous studies examining lipoprotein distribution patterns in response to resistant starch consumption, this endpoint, which is that the MSPrebiotic^{®} Resistant Potato Starch is effective in reducing biomarkers of cardiovascular disease and diabetes, specifically, lipoprotein levels and glucose, is a surprising discovery, as discussed herein.

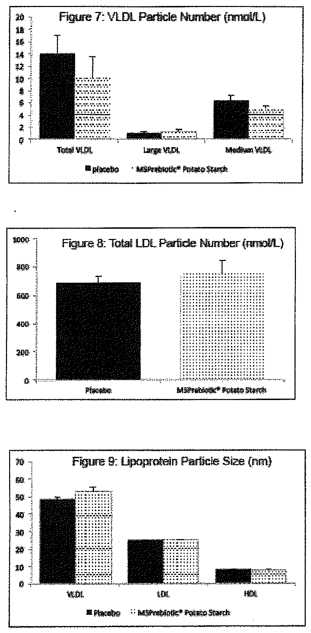

Although traditional cholesterol tests [LDL (bad) and HDL (good) cholesterol)] are the standard indicators of cardiovascular disease risk, a large majority of individuals who have suffered a heart attack have 'normal' cholesterol levels. As it is the lipoproteins that are responsible for carrying cholesterol throughout the body, analysis of lipoprotein particle number and size can provide a more in depth and accurate level of risk analysis. Although VLDL and LDL particle number and size was not different (p<0.05) between the placebo and MSPrebiotic^{®} Resistant Potato Starch groups (Figures 7, 8, 9, Appendix), total HDL particle number was increased by 28% (p<0.05) in response to MSPrebiotic^{®} Resistant Potato Starch pigs versus placebo pigs (Figure 3). An elevated number of HDL lipoprotein particles is considered to be inherently heart healthy as the major function of these lipoproteins is to remove excess cholesterol from peripheral tissues to the liver for removal from the body in a process called 'reverse cholesterol transport'. These results indicate that MSPrebiotic^{®} Resistant Potato Starch is an effective cardioprotective supplement to reduce cardiovascular disease risk by elevating the number of HDL lipoprotein particles.

### Blood Glucose and Insulin

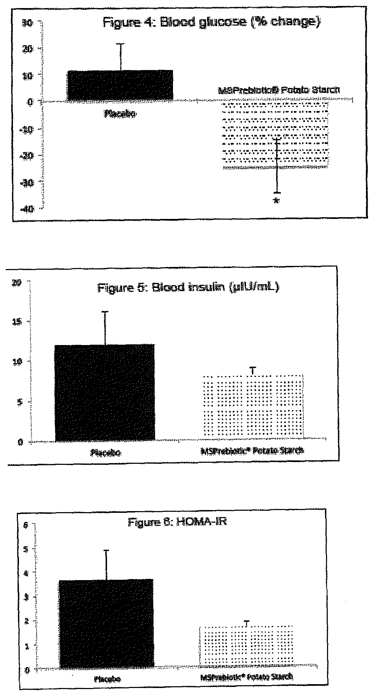

We observed an 11% reduction (p<0.05) in blood glucose following MSPrebiotic^{®} Resistant Potato Starch compared with the placebo capsule group (Figure 4). Similarly, supplementation of MSPrebiotic^{®} Resistant Potato Starch resulted in a numerical reduction in both blood insulin (-31%, p=0.4, Figure 5,) and HOMA-IR (-54%, p=0.14, Figure 6). HOMA-IR is a method to estimate insulin resistance using fasting blood glucose and insulin concentrations. These results suggest that MSPrebiotic^{®} Resistant Starch is an effective dietary supplement to manage blood sugar within a healthy range.

### Implications and Future Opportunities

The results of this pre-clinical evaluation indicate that dietary supplementation of MSPrebiotic^{®} Resistant Potato Starch capsules at a controlled dose of for example 10 g/d is an effective strategy to favorably modulate multiple markers of metabolic syndrome including blood glucose and insulin, as well as HDL particle number.

### Experimental Approach

Twelve 8-week old male Yorkshire pigs were purchased from Michael Fanning Farms (Howe, IN) and treated in accordance with Institutional Animal Care and Use Committee approved guidelines. Pigs were housed individually in an environmentally controlled room at 20° C in research pens fitted with single feeders and drinking nipples within the Animal Care Facility at the University at Buffalo. The animals were allowed free access to water for the duration of the experiment. Animals were fed a grower pig diet for 1 week for acclimation to the surroundings and animal care staff. Following acclimatization, all animals were placed on a high fat/high cholesterol diet meant to reflect standard North American nutrient intakes (Teklad Custom Research Diet, TD.10520, TABLE 1). On day 1 of the experimental period, animals (n=6) were randomly divided into two dietary groups: 1) a placebo group supplemented with capsules containing normal gelatinized starch; and 2) a RS group supplemented with RS capsules containing MSPrebiotic^{®} Resistant Potato Starch. Capsules were prepared at the Richardson Centre for Functional Foods and Nutraceuticals, University of Manitoba, as discussed below. As the capsules were formulated to contain ~0.32g of RS, each animal received ~31 capsules/d mixed in with the morning and evening meals to reach a target RS intake of 10 g/d. Composition of the gelatinized and MSPrebiotic^{®} Resistant Potato Starch is presented in Table 2.

On day 1 of the experimental period each pig was weighed and a fasting blood sample (~5 mls) was obtained from the ear vein into sodium heparin tubes while the pigs were sedated with an intramuscular injection of a swine premix solution [Telazol (100mg/ml) at 4.4-6mg/kg and xylazine (100mg/ml) at 2.2mg/kg].

This blood sample was used as a baseline for all endpoint blood measures. The pigs were fed their respective test diets at a level of roughly 5.5 % of their body weight for the duration of the experiment. Feed intake was monitored on a daily basis while body weights were taken at weekly intervals. On d-30 of the experimental period following an 8-hour overnight fast, the pigs were weighed, sedated with an intramuscular injection of a swine premix solution and subsequently anesthetized with isoflurane (2.5%) in O2 (gas flow rate at 2.5 L/min) for blood collection by cardiac puncture. Following exsanguination, the animals were euthanized by overdose of isoflurane.

### Endpoint analysis:

Plasma total cholesterol (TC), high-density lipoprotein cholesterol (HDL-C), low-density lipoprotein cholesterol (LDL-C), and TAG were determined by automated enzymatic kits on a Pentra 400 autoanalyzer (Kamiya Biomedical Company, Seattle, WA, USA). Direct assessment of lipoprotein particle number and size was conducted by nuclear magnetic resonance spectroscopy (Liposcience, Raleigh, NC). Serum insulin was analysed by ELISA (EZRMI-13K, Millipore, Billerica, MA) and glucose was measured by colorimetric analysis (ab6533, abcam, Cambridge, MA). Insulin resistance was estimated by homeostatic model assessment (HOMA-IR). Blood C-reactive protein (CRP) as measured by ELISA (10011236, Cayman Chemical).

### Statistical Analysis

Data were analyzed with a general linear model ANOVA using experimental block as a fixed factor. Data were analyzed with SPSS 16 for Mac (SPSS Inc, Chicago IL). Data are presented as mean ± SEM. All results are the means from 6 animals. Differences were considered significant at p≤0.05.

Accordingly, the inventors then proceeded to investigate the development of pharmaceutical products.

As discussed herein, considerable care must be taken to ensure that as much of the resistant starch is retained as possible. As discussed below, the inventors have discovered that there are several additional considerations beyond maintaining a temperature below 60°C when preparing pharmaceutical products such as tablets and capsules from resistant starch such as moisture content of the starch and pressure used in tablet formation.

Initially, the inventors attempted to develop resistant starch-containing capsules. However, initial attempts were unsuccessful as the resistant starch tended to clump together and was difficult to fill or flow into a suitably sized capsule. It was subsequently discovered that carefully drying the resistant starch to a moisture content of below 20% for example between 12-19% produced flowable starch that did not stick together. In other embodiments, the moisture content may be below 17%, for example, 12-17% or 12-15%.

Accordingly, in one embodiment of the invention, there is provided a method of preparing a resistant starch capsule comprising drying a quantity of resistant starch to below 20%, for example, below 17% and then flowing the dried resistant starch into a capsule. Specifically, the moisture content may be 12-19%, 12-17% or 12-15%.

The inventors also attempted to prepare resistant starch tablets as there were concerns that the resistant starch capsules may not have been the ideal delivery mechanism due to slow release of the resistant starch from the capsule.

In these embodiments, solution of a suitable excipient is prepared and resistant starch is added to the solution. The mixture is then allowed to form into pellets or granules. The granules are dried and then reduced in size using any suitable means known in the art. The resistant starch material is formed into a tablet under a suitable pressure. Surprisingly, it was found that pressures typically used for tablet preparation, for example 200 - 500 MPa in the preparation of such tablets fractured the granule structure of the resistant starch, thereby greatly reducing the quantity of resistant starch in the tablet. Subsequent experimentation showed that pressures between 60 - 100 MPa is suitable to produce the tablet, while lower pressures produced tablets which broke apart and higher pressures fractured the granule structure of the resistant starch to an unacceptable degree.

In some embodiments, the excipient is a binder, for example, polyvinylpyrrolidone (PVP). Surprisingly, while other binders such as methylcellulose, gelatinized starch and hydroxypropylcelluose were tested, it was discovered that only PVP produced tablets having the desired properties.

In some embodiments, the pharmaceutical composition is prepared as follows: an aqueous solution of 1 part PVP is prepared. 9 parts resistant starch is dissolved therein at a temperature below 60°C. Pellets and granules are allowed to form which are then dried. The dried material is reduced in size with a hammer mill. The material is then formed into a tablet and subjected to a pressure between for example 45-100 MPa or in some preferred embodiments between 60-100 MPa.

As will be appreciated by one of skill in the art, the capsules and tablets may be made in any suitable size, for example, in a unit dosage to be taken once per day, or in dosages to be taken multiple times per day, for example twice or more per day on a suitable dosage regimen or schedule. For example, a suitable dosage regimen may be one or more capsules or tablets comprising 50-750 mg resistant starch prepared as discussed herein every 2, 4, 6, 8, 12 or 24 hours or taken with meals.

For example, the capsules or tablets may be 50 mg, 100 mg, 200 mg, 220 mg, 250 mg, 300 mg, 350 mg, 400 mg, 450 mg, 500 mg, 600 mg, 700 mg, 750 mg or any suitable similar size according to patient and/or consumer preference.

In some embodiments, each capsule may weigh 625 ± 5.0 mg and each capsule may contain 528 ± 17.6 mg of material of which 350-370 mg is resistant starch.

In some embodiments, the material is formed into tablets at a pressure between 45-100 MPa or between 60-100 MPa. In some embodiments, each tablet is 40-50% resistant starch, for example, 45% resistant starch.

In one embodiment of the invention, there is provided a method of preparing a resistant starch pharmaceutical composition comprising mixing an effective amount of resistant starch with a suitable excipient. The excipient may be PVP. The mixture may be 1 part PVP to 9 parts resistant starch or resistant starch source. In some embodiments, the pharmaceutical composition is in the form of a tablet. In other embodiments, resistant starch is dried to a moisture content below 20% for example between 12-19% or below 17% for example between 12-17% and flowed into a suitably sized capsule, thereby producing a resistant starch capsule.

As will be readily apparent to one of skill in the art, "an effective amount" will depend on the animal, its age, weight and general condition, among other factors. However, as discussed above, the inventors have discovered that a much lower level of resistant starch than previously believed is sufficient to treat or otherwise ameliorate at least one of the symptoms associated with infectious diarrhea, postweaning diarrhea and/or gastrointestinal stresses associated with weaning such as fecal consistency, daily food intake and the like. For example, in some embodiments, the "effective amount" is resistant starch at approximately 0.1 %-2.5%, 0.1-2.0%, 0.1-1.5%, 0.1-1.0%, 0.2-2.5%, 0.2-2.0%, 0.2-1.5%, 0.2-1.0%, 0.3-2.5%. 0.3-2.0%, 0.3-1.5%, 0.3-1.0%, 0.4-2.5%. 0.4-2.0%, 0.4-1.5%, 0.4-1.0%, 0.5-2.5%. 0.5-2.0%, 0.5-1.5%, or 0.5-1.0% of the animal's diet.

In other embodiments, the "effective amount" may be a resistant starch capsule or tablet. The resistant starch capsule or tablet may be prepared according to the methods described herein. Preferably, the resistant starch capsule is a 500 mg capsule. The tablet may be a 220 mg tablet or a 250 mg tablet.

### PREPARATION OF RESISTANT STARCH TABLETS

Formulation: 90 % resistant starch + 10 % PVP, the water should be 30% of the total amount of the flour. For example, 9 kg resistant starch plus 1 kg PVP, the water should be 10×30%=3kg (3 liter).

### Procedure

- Step 1: Resistant starch, PVP, and water are weighed respectively.
- Step 2: Dissolve the PVP into water, heat and dissolve it.
- Step 3: Cool down the PVP solution in ice water bath into room temperature.
- Step 4: Add the PVP solution into Resistant starch powders and mix thoroughly with a mixer (10 - 20 min depending on the amount).
- Step 5: Screening the damp mass through a mesh to form pellets or granules with a granulator.
- Step 6: Drying the granules by using a dryer in 40-45 °C for about 72h, depending on the amount of the granules prepared.
- Step 7: After the granules are dried, they are passed through a hammer mill (We use #4 screen, with 0.75mm diameter holes).
- Step 8: Go through the tablet machine (Pressure of 60 - 100 MPa is required for the resistant starch which contains 60 - 75% RS. Higher RS requires higher pressure. The final tablet product should contain about 40% RS, db).

The scope of the claims should not be limited by the preferred embodiments set forth in the examples, but should be given the broadest interpretation consistent with the description as a whole.

**TABLE 1**

| **Ingredient** | **g.kg** |
|---|---|
| **Casein** | 214.0 |
| DL-Methionine | 2.5 |
| Sucrose | 300.0 |
| **Maltodextrin** | 215.7 |
| Lard | 150.0 |
| Cholesterol | 15.0 |
| Cellulose | 25.8 |
| Vitamin Mix, AIN-93-VX (94047) | 15.0 |
| **Choline Bitartrate** | 4.5 |
| TBHQ, antioxidant | 0.01 |
| Calcium Phosphate, dibasic | 20.0 |
| Calcium Carbonate | 8.9 |
| Potassium Citrate, monohydrate | 12.84 |
| Other | 15.58 |
| **Nutrient Composition (% energy)** | 49.5 |
| Carbohydrates | 18.0 |
| Protein | 32.5 |
| Fat | |

**Table 2 Characterization of starches used in the feeding study**

| **Item** | **Gela)nized Starch (Placebo)** | **MSPrebio)c^{®} Potato Starch** |
|---|---|---|
| Resistant starch (%) | 0 | 60 |
| Total starch per capsule (g) | 0.44 | 0.53 |
| Resistant starch (g/capsule) | 0 | 0.32 |
| Readily available starch (g) | 0.44 | 0.21 |

### REFERENCES

1. Robertson MD: Dietary-resistant starch and glucose metabolism. Curr Opin Clin Nutr Metab Care 2012, 15(4):362-367.
2. Aller EE, Abete I, Astrup A, Martinez JA, van Baak MA: Starches, sugars and obesity. Nutrients 2011, 3(3):341-369.
3. Bhandari SK, Nyachoti CM, Krause DO: Raw potato starch in weaned pig diets and its influence on postweaning scours and the molecular microbial ecology of the digestive tract. J Anim Sci 2009, 87(3):984-993.
4. Kendall CW, Emam A, Augustin LS, Jenkins DJ: Resistant starches and health. J AOAC Int 2004, 87(3):769-774.
5. Rideout TC: Getting personal: considering variable interindividual responsiveness to dietary lipid-lowering therapies. Curr Opin Lipidol 2011, 22(1):37-42.
6. Rideout, TC et al. Nutrient utilisation and intestinal fermentation are differentially affected by the consumption of resistant starch varieties and conventional fibres in pigs. British Journal of Nutrition 2008, 99:984-992.

### Group Meansand Statistical Analysis

### Group Statistics

| | diet | N | Mean | Std. Deviation | Std. Error Mean |
|---|---|---|---|---|---|
| VLOLP | 1.00 | 6 | 14.0167 | 7.01331 | 2.86317 |
| | 2.00 | 5 | 10.1860 | 7.41435 | 3.31580 |
| LOLP | 1.00 | 6 | 687.9400 | 121.58705 | 49.63770 |
| | 2.00 | 6 | 750.7967 | 225.26834 | 91.96541 |
| HOLP | 1.00 | 6 | 50.6000 | 11.19202 | 4.56912 |
| | 2.00 | 5 | 64.8920 | 5.73582 | 2.56514 |
| VLOLsize | 1.00 | 6 | 48.4850 | 5.96435 | 2.43494 |
| | 2.00 | 6 | 52.7917 | 5.32634 | 2.17447 |
| LDLsize | 1.00 | 6 | 24.9750 | .02881 | .01176 |
| | 2.00 | 6 | 24.9767 | .04761 | .01944 |
| HOLsize | 1.00 | 6 | 8.1717 | .34067 | .13908 |
| | 2.00 | 6 | 7.9583 | .15211 | .06210 |
| calTAG | 1.00 | 6 | 33.3383 | 9.35784 | 3.82032 |
| | 2.00 | 5 | 38.3740 | 9.79292 | 4.37953 |
| caIVLOLTAG | 1.00 | 6 | 13.6800 | 7.80738 | 3.18735 |
| | 2.00 | 5 | 16.5080 | 6.59142 | 2.94777 |
| caIHOLC | 1.00 | 6 | 56.5 300 | 12.33928 | 5.03749 |
| | 2.00 | 6 | 55.6150 | 10,82422 | 4.41897 |
| glucose | 1.00 | 6 | 114.0000 | 2 3.15167 | 9.45163 |
| | 2.00 | 5 | 89.6000 | 2 5.74490 | 11.51347 |
| insulin | 1.00 | 6 | 11.8333 | 10.68095 | 4.36048 |
| | 2.00 | 5 | 3.1600 | 1.67720 | .75007 |
| totalC | 1.00 | 6 | 209.1667 | 33.33117 | 13.60739 |
| | 2.00 | 6 | 225.0000 | 33.38263 | 13.62840 |
| HOLC | 1.00 | 6 | 58.3333 | 14.54189 | 5.93670 |
| | 2.00 | 6 | 54.6667 | 7.03325 | 2.87131 |
| LOIdirect | 1.00 | 6 | 201.5000 | 32.07959 | 13.09644 |
| | 2.00 | 6 | 248.6667 | 72.18495 | 29.46938 |
| LOIbase | 1.00 | 4 | 40.5000 | 29.03446 | 14.51723 |
| | 2.00 | 3 | 51.0000 | 12.49000 | 7.21110 |
| LOIchange | 1.00 | 4 | 235.1025 | 171.84862 | 85.92431 |
| | 2.00 | 3 | 399.8700 | 73.56742 | 42.47417 |
| glucosebase | 1.00 | 4 | 86.7500 | 58.32881 | 29.16440 |
| | 2.00 | 3 | 140.0000 | 18.02776 | 10.40833 |
| glucsosechange | 1.00 | 4 | 8.3025 | 12.19190 | 6.09595 |
| | 2.00 | 3 | -24.8567 | 17.94504 | 10.36057 |
| insulinbase | 1.00 | 4 | 10.7750 | 10.02576 | 5.01288 |
| | 2.00 | 3 | 21.5333 | 21.07637 | 12.16845 |
| insulinchange | 1.00 | 4 | -2.1525 | 51.99271 | 25.99635 |
| | 2.00 | 3 | -36.5333 | 48.98915 | 28.28390 |
| HOMAR | 1.00 | 5 | 3.6560 | 2.73476 | 1.22302 |
| | 2.00 | 5 | 1.6780 | .45598 | .20392 |

### Independent Samples Test

| | | Levene's Test for Equality of **Variances** | | t-test for Equality of Means | | | | | | |
|---|---|---|---|---|---|---|---|---|---|---|
| | | F | Sig. | t | df | Sig. (2-tailed) | **Mean** Difference | Std. Errnr Difference | 9S% Confidence Interval of the Difference | |
| | | | | | | | | | **Lower** | Upper |
| VLDLP **assumed** | **Equalvariances** | .160 | .698 | 879 | 9 | .402 | 3.83067 | 4.3S637 | -6.02414 | 13.68S47 |
| **Equalvariances not assumed** | | | | 874 | 8.437 | .406 | 3.83067 | 4.38090 | -6.18144 | 13.84277 |
| LDLP **assumed** | **Equalvarlances** | 3.402 | .095 | -.601 | 10 | 561 | -62.85667 | 104.50617 | -295.71092 | 169.99758 |
| **Equalvariances not assumed** | | | | -.601 | 7.685 | 565 | -62.85667 | 104.50617 | -305.57672 | 179.86338 |
| HDLP **assumed** | **Equaivariances** | 3.599 | .090 | -2.572 | 9 | 030 | -14.29200 | 555676 | -26.86227 | -1.72173 |
| **Equalvariances not assumed** | | | | -2.728 | 7.693 | 027 | -1429200 | 5.23993 | -26.45969 | -2.12431 |
| VLDLsize | **Equalvarlances** | .165 | 694 | -1.319 | 10 | 217 | -4.30667 | 3.26454 | -11.58052 | 2.96719 |
| **Equalvariances not assumed** | | | | -1.319 | 9.875 | 217 | -4.30667 | 3.26454 | -11.59305 | 2.97972 |
| LDLsize **assumed** | Equal variances | 396 | 543 | -.073 | 10 | 943 | -.00167 | .02272 | -.05229 | 04895 |
| **Equalvariances not assumed** | | | | -.073 | 8.229 | 943 | -.00167 | .02272 | -.05380 | .05047 |
| HDLsize **assumed** | **Equalvariances** | 4.246 | .066 | 1.401 | 10 | 192 | 2 1333 | .15231 | -.12604 | .55271 |
| **Equalvariances not assumed** | | | | 1.401 | 6.917 | 205 | 2 1333 | .15231 | -.14770 | .57437 |
| calTAG **assumed** | **Equalvariances** | .009 | 925 | -.870 | 9 | .407 | -5.03567 | 5.78502 | -18.122 30 | 8.05097 |
| **Equalvarlances not assumed** | | | | -866 | 8.477 | .410 | -5.03567 | 5.81164 | -18.30721 | 8.23588 |
| calVLDLTAG **assumed** | **Equalvariances** | 987 | 346 | -.640 | 9 | 538 | -2.82800 | 4.41555 | -12.81666 | 7.16066 |
| **Equalvariances not assumed** | | | | -.65 ■ | 8.990 | 531 | -2.82800 | 4.34150 | -12.65081 | 6.99481 |
| calHDLC **assumed** | **Equalvariances** | 413 | .535 | 137 | 10 | .894 | .91500 | 6.70101 | -14.01579 | 15.84579 |
| **Equalvariances not** | **assumed** | | | .137 | 9.833 | 894 | .91500 | 6.70101 | -14.05020 | 15.88020 |
| glucose | **Equalvariances assumed** | 038 | .849 | 1.656 | 9 | 132 | 2 4.40000 | 14.73761 | -8.93878 | 57.73878 |
| **Equalvarlances not assumed** | | | | 1.638 | 8.221 | 139 | 2 4.40000 | 14.89608 | -9.79036 | 58.59036 |
| **nsulln assumed** | **Equalvariances** | 4.764 | .057 | 755 | 9 | .470 | 3.67333 | 4.86801 | -7.33886 | 14.68553 |
| **Equalvarlances not assumed** | | | | 830 | 5.294 | 442 | 3.67333 | 4.42452 | -7.51260 | 14.85926 |
| totalC **assumed** | **Equalvariances** | 169 | .689 | -.822 | 10 | .430 | -15.83333 | 1925862 | -58.7442 1 | 27.07755 |
| **Equalvarlances not assumed** | | | | -.822 | 10.000 | .430 | -15.83333 | 1925862 | -58.74423 | 27.07756 |
| HDLC **assumed** | **Equalvariances** | 1.107 | 318 | .556 | 10 | .590 | 3.66667 | 6.59461 | -11.02704 | 18.36037 |
| **Equalvariances not assumed** | | | | 556 | 7.218 | 595 | 3.66667 | 6.59461 | -11.83220 | 19.16553 |
| LDLdirect **assumed** | **Equalvariances** | 5.198 | .046 | -1.463 | 10 | 174 | -47.16667 | 32.24843 | -119.02064 | 24.68731 |
| **Equalvarlances not assumed** | | | | -1.463 | 6.901 | 188 | -47.16667 | 32.24843 | -123.64476 | 29.31143 |
| LDLbase **assumed** | **Equalvariances** | 1.430 | 285 | -.577 | 5 | 589 | -10.50000 | 18.20577 | -5729942 | 36.2 994 2 |
| **Equalvariances not assumed** | | | | -.648 | 4.273 | 550 | -10.50000 | 1620957 | -54.39384 | 33.39384 |
| LDL change **assumed** | **Equalvariances** | 1.695 | 250 | -1.530 | 5 | 187 | -164.76750 | 107.69875 | -441.61595 | 112.08095 |
| **Equalvarlances not assumed** | | | | -1.719 | 4.263 | .156 | -164.76750 | 95.84906 | -424.52671 | 94.99171 |
| glucosebase **assumed** | **Equalvarlances** | 2.765 | .157 | -1.496 | 5 | .195 | -53.25000 | 35.58962 | -144.73602 | 38.23602 |
| **Equalvarlances not assumed** | | | | -1.720 | 3.722 | 166 | -53.25000 | 30.96604 | -141.81528 | 35.31528 |
| glucsosechange **assumed** | **Equalvariances** | 887 | 389 | 2.941 | 5 | 032 | 33.15917 | 1127670 | 4.17150 | 62.14684 |
| **Equalvariances not assumed** | | | | 2 .758 | 3.356 | .062 | 33.15917 | 12.02 090 | -2.89877 | 69.21711 |
| insulinbase **assumed** | **Equalvariances** | 4.399 | .090 | -913 | 5 | .403 | -10.75833 | 11.78263 | -41.04654 | 19.52988 |
| **Equalvariances not assumed** | | | | -.817 | 2.685 | .480 | -10.75833 | 13.16055 | 55.56406 | 34.04739 |
| **insulinchange** | **Equalvariances assumed** | 000 | 999 | 886 | 5 | 416 | 34.38083 | 38.80876 | -65.38026 | 134.14193 |
| **Equalvariances not** | assumed | | | 895 | 4.612 | 415 | 34.38083 | 38.41601 | -66.92160 | 135.68327 |
| HOMAIR **assumed** | **Equalvariances** | 5.905 | .041 | 1.595 | 8 | 149 | 1.97800 | 1.23991 | -.88123 | 4.83 723 |
| **Equalvarlances not assumed** | | | | 1.595 | 4.222 | 182 | 1.97800 | 1.23991 | -1.39424 | 5.35024 |

## Claims

1. Native, unmodified RS type 2 resistant potato starch having a resistant starch content of at least 60% for use in the treatment of a human for one of the following:
a) increasing High Density Lipoprotein plasma levels,
b) decreasing blood glucose levels,
c) decreasing insulin resistance,
comprising administering to said human an effective amount of resistant potato starch on a regular schedule or regimen, wherein the effective amount is between 0.25 and 40 g, preferably between 0.5 and 40 g and more preferably between 0.5 and 30 g.

2. Resistant potato starch for use according to claim 1, wherein the effective amount is between 0.1% and 2.5% of the individuals diet.

3. Resistant potato starch for use according to claim 1, wherein the effective amount is 10g per day.

4. Resistant potato starch for use according claim 1, wherein the resistant starch is kept during production of the resistant starch and preparation of medicaments, functional foods or beverages at a temperature below 60°C.

5. Resistant potato starch for use according to any of the above claims, wherein the human in need for the resistant starch treatment exhibits one of the following:
a) Human with a fasting HDL plasma level below 40 mg/dl or below 1 mmol/l,
b) Human with a fasting LDL to HDL ratio of greater than 5,
c) Human with a high fasting LDL levels combined with low fasting HDL levels;
d) Human with familial history of cardiovascular disease,
e) Human otherwise considered to be at risk of developing cardiovascular disease,
f) Human with blood glucose levels of greater than 100mg/dL,
g) Human having diabetes,
f) Human having, preferably chronic, hyperglycemia,
g) Human with Type 2 diabetes,
h) Human with a familial history of Type 2 diabetes,
i) Human at risk of developing Type 2 diabetes.

6. Resistant potato starch for use according to any of the above claims, wherein the resistant starch is used for preparation of medicaments, functional foods or beverages.

7. Resistant potato starch for use according to any of the above claims, wherein the starch is administered as a powder or encapsulated in capsules.

8. Pharmaceutical composition comprising resistant potato starch for use according to any of the above claims and a suitable excipient, being preferably Polyvinylpyrrolidon.

9. Pharmaceutical composition according to claim 8, wherein the resistant potato starch for use is dried to a moisture content below 20%, preferably below 17%.

## Patentansprüche

1. Native, unmodifizierte resistente Kartoffelstärke RS Typ 2 mit einem Gehalt an resistenter Stärke von mindestens 60 % zur Verwendung bei der Behandlung eines Menschen für Folgendes:
a) Erhöhung der High-Density-Lipoprotein-Plasmaspiegel,
b) Senkung des Blutzuckerspiegels,
c) Verringerung der Insulinresistenz,
einschließlich der Verabreichung einer wirksamen Menge von resistenter Kartoffelstärke an besagten Menschen in einem regelmäßigen Zeitplan oder Schema, wobei die wirksame Menge zwischen 0,25 und 40 g, vorzugsweise zwischen 0,5 und 40 g und noch besser zwischen 0,5 und 30 g liegt.

2. Resistente Kartoffelstärke zur Verwendung gemäß Anspruch 1, wobei die wirksame Menge zwischen 0,1 % und 2,5 % der Ernährung des Individuums liegt.

3. Resistente Kartoffelstärke zur Verwendung gemäß Anspruch 1, wobei die wirksame Menge 10 g pro Tag beträgt.

4. Resistente Kartoffelstärke zur Verwendung gemäß Anspruch 1, wobei die resistente Stärke während der Herstellung der resistenten Stärke und der Zubereitung von Medikamenten, funktionellen Lebensmitteln oder Getränken bei einer Temperatur unter 60 °C gehalten wird.

5. Resistente Kartoffelstärke zur Verwendung gemäß einem der obigen Ansprüche, wobei der Mensch, für den die Behandlung mit resistenter Stärke erforderlich ist, eines der folgenden Merkmale aufweist:
a) Mensch mit einem HDL-Plasmaspiegel in nüchternem Zustand von unter 40 mg/dl oder unter 1 mmol/l,
b) Mensch mit einem LDL-HDL-Quotienten in nüchternem Zustand von über 5,
c) Mensch mit einem hohen LDL-Spiegel in nüchternem Zustand in Kombination mit einem niedrigen HDL-Spiegel in nüchternem Zustand;
d) Mensch mit einer familiären Vorgeschichte von Herz-Kreislauf-Erkrankungen,
e) Mensch, der anderweitig als gefährdet gilt, Herz-Kreislauf-Erkrankungen zu entwickeln,
f) Mensch mit einem Blutzuckerspiegel von über 100 mg/dL,
g) Mensch mit Diabetes,
f) Mensch mit, vorzugsweise chronischer, Hyperglykämie,
g) Mensch mit Typ-2-Diabetes,
h) Mensch mit einer familiären Vorgeschichte von Typ-2-Diabetes,
i) Mensch, der gefährdet ist, eine Typ-2-Diabetes zu entwickeln.

6. Resistente Kartoffelstärke zur Verwendung gemäß einem der obigen Ansprüche, wobei die resistente Stärke zur Herstellung von Arzneimitteln, funktionellen Lebensmitteln oder Getränken verwendet wird.

7. Resistente Kartoffelstärke zur Verwendung gemäß einem der obigen Ansprüche, wobei die Stärke als Pulver oder eingekapselt in Kapseln verabreicht wird.

8. Pharmazeutische Zusammensetzung, die resistente Kartoffelstärke zur Verwendung gemäß einem der obigen Ansprüche und einen geeigneten Hilfsstoff, vorzugsweise Polyvinylpyrrolidon, enthält.

9. Pharmazeutische Zusammensetzung gemäß Anspruch 8, wobei die zu verwendende resistente Kartoffelstärke auf einen Feuchtigkeitsgehalt von unter 20 %, vorzugsweise unter 17 %, getrocknet ist.

## Revendications

1. Amidon de pomme de terre résistant AR non modifié natif de type 2 ayant une teneur en amidon résistant d'au moins 60 % destiné au traitement d'un humain dans l'un des cas suivants :
a) augmentation des taux plasmatiques de lipoprotéine de haute densité,
b) diminution des taux de glycémie,
c) diminution de la résistance à l'insuline,
comprenant l'administration audit humain d'une quantité efficace d'amidon de pomme de terre résistant dans un programme ou régime régulier, dans lequel la quantité efficace est comprise entre 0,25 et 40 g, de préférence entre 0,5 et 40 g et, de façon plus préférentielle, entre 0,5 et 30 g.

2. Amidon de pomme de terre résistant pour l'utilisation selon la revendication 1, dans lequel la quantité efficace est comprise entre 0,1 % et 2,5 % du régime de l'individu.

3. Amidon de pomme de terre résistant pour l'utilisation selon la revendication 1, dans lequel la quantité efficace est de 10 g par jour.

4. Amidon de pomme de terre résistant pour l'utilisation selon la revendication 1, dans lequel l'amidon résistant est maintenu à une température inférieure à 60 °C pendant la production de l'amidon résistant et la préparation des médicaments, des aliments ou boissons fonctionnels.

5. Amidon de pomme de terre résistant pour l'utilisation selon l'une quelconque des revendications ci-dessus, dans lequel l'humain ayant besoin du traitement par amidon résistant présente une des caractéristiques suivantes :
a) Humain présentant un taux plasmatique de HDL à jeun inférieur à 40 mg/dl ou inférieur à 1 mmol/l,
b) Humain présentant un rapport LDL/HDL à jeun supérieur à 5,
c) Humain présentant des taux de LDL à jeun élevés associés à de faibles taux de HDL à jeun ;
d) Humain présentant des antécédents familiaux de maladie cardiovasculaire,
e) Humain par ailleurs considéré comme étant à risque de développer une maladie cardiovasculaire,
f) Humain présentant des taux de glycémie supérieurs à 100 mg/dL,
g) Humain souffrant de diabète,
f) Humain souffrant d'hyperglycémie, de préférence chronique,
g) Humain souffrant de diabète de type 2,
h) Humain présentant des antécédents familiaux de diabète de type 2,
i) Humain à risque de développer un diabète de type 2.

6. Amidon de pomme de terre résistant pour l'utilisation selon l'une quelconque des revendications ci-dessus, dans lequel l'amidon résistant est utilisé pour la préparation de médicaments, d'aliments ou de boissons fonctionnels.

7. Amidon de pomme de terre résistant pour l'utilisation selon l'une quelconque des revendications ci-dessus, dans lequel l'amidon est administré sous forme de poudre ou encapsulé dans des gélules.

8. Composition pharmaceutique comprenant l'amidon de pomme de terre résistant pour l'utilisation selon l'une quelconque des revendications ci-dessus et un excipient approprié, étant de préférence du polyvinylpyrrolidone.

9. Composition pharmaceutique selon la revendication 8, dans laquelle l'amidon de pomme de terre résistant pour l'utilisation est séché jusqu'à une teneur en humidité inférieure à 20 %, de préférence inférieure à 17 %.
